Europäisches Patentamt

European Patent Office

Office européen des brevets

(19)

(11) **EP 1 207 892 B1**

(12) **FASCICULE DE BREVET EUROPEEN**

(45) Date de publication et mention
de la délivrance du brevet:
**20.10.2004 Bulletin 2004/43**

(21) Numéro de dépôt: **00960776.3**

(22) Date de dépôt: **01.09.2000**

(51) Int Cl.7: **A61K 35/56**, A61K 38/01,
A61K 7/48

(86) Numéro de dépôt international:
**PCT/FR2000/002420**

(87) Numéro de publication internationale:
**WO 2001/017538 (15.03.2001 Gazette 2001/11)**

(54) **UTILISATION D'HYDROLYSATS ENZYMATIQUES DE CHAIR D'HUITRES POUR LA PREPARATION DE COMPOSITIONS ANTIRADICALAIRES**

VERWENDUNG VON ENZYMATISCHEN AUSTERFLEISCHHYDROLYSATEN ZUR HERSTELLUNG VON ANTIRADIKALISCHEN ZUSAMMENSETZUNGEN

USE OF OYSTER FLESH ENZYMATIC HYDROLYSATES FOR PREPARING COMPOSITIONS ELIMINATING FREE RADICALS

(84) Etats contractants désignés:
**AT BE CH CY DE DK ES FI FR GB GR IE IT LI LU MC NL PT SE**

(30) Priorité: **03.09.1999 FR 9911060**

(43) Date de publication de la demande:
**29.05.2002 Bulletin 2002/22**

(73) Titulaires:
• **IFREMER
INSTITUT FRANCAIS DE RECHERCHE POUR L'EXPLOITATION DE LA MER
92138 Issy-les-Moulineaux Cédex (FR)**
• **ASSISTANCE PUBLIQUE, HOPITAUX DE PARIS
75004 Paris (FR)**

(72) Inventeurs:
• **DURAND, Patrick
F-44400 Reze (FR)**
• **LANDREIN, Annie
F-44300 Nantes (FR)**
• **ROY, Philippe
F-44000 Nantes (FR)**
• **LINDENBAUM, Albert
F-75020 Paris (FR)**
• **EDEAS, Marvin
F-75013 Paris (FR)**

(74) Mandataire: **Goulard, Sophie et al
Cabinet ORES,
36,rue de St Pétersbourg
75008 Paris Cedex (FR)**

(56) Documents cités:
• **DATABASE WPI Section Ch, Week 197413 Derwent Publications Ltd., London, GB; Class D12, AN 1974-23535V XP002140617 & JP 48 022661 A (MATSUKURA K), 23 mars 1973 (1973-03-23)**
• **DATABASE WPI Section Ch, Week 199521 Derwent Publications Ltd., London, GB; Class B04, AN 1995-158876 XP002140618 & JP 07 082132 A (MIKIMOTO SEIYAKU KK), 28 mars 1995 (1995-03-28)**
• **PATENT ABSTRACTS OF JAPAN vol. 1995, no. 07, 31 août 1995 (1995-08-31) & JP 07 102252 A (MIKIMOTO PHARMACEUT CO LTD), 18 avril 1995 (1995-04-18)**
• **DATABASE WPI Section Ch, Week 199846 Derwent Publications Ltd., London, GB; Class B04, AN 1998-537367 XP002140619 & JP 10 236941 A (NOEVIR KK), 8 septembre 1998 (1998-09-08)**
• **DATABASE WPI Section Ch, Week 200036 Derwent Publications Ltd., London, GB; Class B04, AN 2000-420753 XP002153646 & NZ 329 018 A (IND RES LTD), 28 avril 2000 (2000-04-28)**
• **JP 07 102252 A (MIKIMOTO PHARMACEUT CO LTD), 18 avril 1995 (1995-04-18) - computer translation**

**Description**

La présente Invention se rapporte à l'utilisation d'hydrolysats enzymatiques de chair d'huitres pour la préparation de compositions antiradicalaires, utiles notamment en thérapeutique, en diététique et en cosmétologie.

Les radicaux libres oxygénés sont des atomes ou des molécules qui possèdent un électron non apparié au niveau de leur orbitale externe (OH$^-$, O$_2^-$, ROO$^-$, RO$^-$,...). De ce fait, ils sont extrêmement instables et peuvent réagir avec des molécules stables telles que des lipides, des glucides, des protéines ou des acides nucléiques, éléments fondamentaux des cellules, pour apparier leur électron, réaction qui conduit à la formation en chaîne de nouveaux radicaux libres. Aussi, sont-ils susceptibles de provoquer de graves altérations cellulaires telles qu'une mutation ou un vieillissement cellulaire, voire la mort des cellules.

Au niveau cellulaire, des radicaux libres oxygénés se forment en permanence. Ils peuvent également se former au cours des mécanismes de détoxication après exposition à certaines substances ou sous l'effet de radiations. Normalement, la production endogène de radicaux libres oxygénés est contrebalancée par la présence de systèmes de défense représentés, d'une part, par des enzymes (superoxyde dismutases; catalases, glutathion peroxydases) qui interceptent les formes actives de l'oxygène, et, d'autre part, par des *"piégeurs de radicaux libres"* (glutathion, acide urique, vitamine C, vitamine A, vitamine E, taurine, ...) qui bloquent les peroxydations en chaîne des lipides membranaires. en sorte que les organismes n'ont pas à en souffrir.

Cependant, de nombreuses situations peuvent entraîner la formation excessive de radicaux libres oxygénés : l'exposition intense au soleil, l'intoxication par certains produits chimiques et certains médicaments, l'hyperoxygénation ou la réoxygénation brutale de tissus préalablement privés d'oxygène, la survenue d'une réaction inflammatoire intense (brûlures, infections, ...) ou chronique. Un excès de radicaux libres oxygénés peut également être lié à une maladie génétique ou à une diminution des défenses : immaturité des systèmes enzymatiques chez les nouveau-nés, vieillissement, déficits alimentaires en vitamines et en oligo-éléments (sélénium, zinc, ...).

Quoi qu'il en soit, on impute à un déséquilibre entre la production et la destruction des radicaux libres oxygénés une responsabilité dans la genèse et l'entretien d'un certain nombre de pathologies chroniques telles que l'athérosclérose, les affections malignes, les pathologies inflammatoires (maladie de Crohn par exemple) et neurodégénératives (maladie d'Alzheimer, maladie de Parkinson, ...) ou encore le vieillissement, ainsi que de pathologies aiguës comme les lésions de reperfusion post-ischémique, les brûlures, les chocs septiques, les infections virales, les états infectieux graves et les allergies, sans qu'il soit toujours possible de préciser si ces radicaux libres sont la cause ou la conséquence, ou les deux simultanément, de la maladie. On comprend, de ce fait, que de très nombreux travaux visent actuellement à mieux appréhender l'implication des radicaux libres oxygénés en physiopathologie et à développer des composés ou compositions propres à s'opposer aux effets délétères de ces radicaux libres.

Des Auteurs (LIVINGSTONE et *al*., 1990, *Funct. Ecol*., 4, 415-424 ; REGOLI et PRINCIPATO, 1995, *Aquat. Tox*., 31, 143-164) ont mis en évidence chez les mollusques marins, non seulement la présence de superoxydes dismutases, de catalases et de glutathion peroxydases, mais également celle d'enzymes anti-oxydantes spécifiques comme la glyoxalase qui catalyse la détoxification des cétoaldéhydes formés au cours du stress oxydatif, et les glutathion transférases qui catalysent une grande variété de réactions de conjugaison du glutathion avec des composés xénobiotiques, signant une aptitude de ces organismes à se protéger contre les radicaux libres oxygénés. Par ailleurs, des anti-oxydants comme le glutathion, la vitamine A, la vitamine E et la taurine, ont été détectés chez les mollusques marins, et se sont montrés dans certains cas être quantitativement proportionnels au stress oxydatif subi par ces animaux.

Aussi, est-il apparu que les mollusques marins étaient susceptibles de constituer une source de composés antiradicalaires utilisables dans la prévention et le traitement des effets néfastes des radicaux libres oxygénés.

Un certain nombre d'Auteurs se sont plus spécialement intéressés aux potentiels antiradicalaires d'extraits d'huîtres. Notamment :

- TAPIERO et TEW (*Biomed. & Pharmacother*., 1996, 50, 149-153) ont étudié les effets d'un lyophilisat d'huîtres dénommé JCOE (JAPAN CLINIC Oyster Extract) sur la teneur intracellulaire en hormone stimulant le glutathion (GSH), ainsi que sur l'activité de la glutathion-S-transférase (GST), d'une culture de cellules HL60. Ce lyophilisat est obtenu en chauffant à 80°C pendant 1 heure de la chair d'huîtres, puis en soumettant le produit résultant à une centrifugation et en lyophilisant le surnageant ainsi recueilli. TAPIERO et TEW ont ainsi mis en évidence une augmentation significative de la synthèse de la GSH chez les cellules HL60 cultivées en présence du lyophilisat, sans toutefois noter de modification significative de l'activité de la GST.

- YOSHIKAWA et *al. (Biomed. & Pharmacother*., 1997, 51, 328-332) ont montré qu'un lyophilisat d'huîtres JCOE est capable *in vitro* de piéger les radicaux superoxydes et hydroxyles, et de protéger des cellules de la muqueuse gastrique de rats contre les effets délétères du peroxyde d'hydrogène, lorsque ces cellules sont prétraitées pendant 24 heures par ce lyophilisat.

- KIMURA et *al. (Journal of Ethnopharmacology,* 1998, 59, 117-123) ont montré que des rats nourris avec de l'huile de maïs peroxydée et recevant, deux fois par jour et par voie orale, un extrait aqueux d'huîtres, présentent des taux sériques en acides gras libres, triglycérides et peroxydes lipidiques, et un taux hépatique en cholestérol total moins élevés que ceux observés chez des rats nourris de la même façon mais ne recevant pas d'extrait aqueux d'huîtres. Par ailleurs, ces Auteurs ont mis en évidence la présence, dans cet extrait aqueux, d'une substance capable à la fois d'inhiber la lipolyse induite par l'adrénaline et de stimuler la lipogenèse à partir du glucose dans des cellules adipeuses de rats, et qu'ils ont identifiée comme étant de l'adénosine.

- NOMURA et *al.* ont proposé, dans la Demande de Brevet Européen publiée sous le n° 0 806 465 au nom de JAPAN CLINIC Co. Ltd, de préparer une composition anti-oxydante par un procédé consistant à fractionner par de l'éthanol un extrait aqueux d'huîtres préalablement obtenu en chauffant un mélange de chair d'huîtres et d'eau à une température comprise entre 50 et 90°C pendant 2 à 3 heures. Les propriétés anti-oxydantes de la composition ainsi préparée sont mises en évidence, dans cette Demande de Brevet, par le biais de tests visant à apprécier son aptitude à inhiber *in vitro* la réaction entre des anions superoxydes produits par un système enzymatique xanthine-xanthine oxydase et le 5,5-diméthyl-1-pyrrolen-1-oxyde.

- DUSSART *(Rapport IFREMER : Stage de VIème Année,* 1997, Faculté de Pharmacie, UNIVERSITE DE LILLE II) a réalisé une étude visant à comparer les propriétés antiradicalaires d'extraits aqueux d'huîtres préparés en mélangeant un broyat de chair d'huîtres avec de l'eau désionisée, puis en soumettant le mélange résultant à une centrifugation suivie d'une lyophilisation du surnageant, avec celles présentées par des extraits d'huîtres préparés en soumettant un broyat d'huîtres uniquement à une lyophilisation. Les résultats de cette étude montrent que, si les deux types d'extraits d'huîtres ont *in vitro* un effet protecteur contre les oxydations induites d'une part, par un générateur de radicaux peroxydes sur des hématies, et, d'autre part, par le cuivre sur les lipoprotéines de basse densité (LDL), les extraits aqueux d'huîtres apparaissent présenter le potentiel anti-oxydant le plus intéressant.

Il a par ailleurs été proposé, dans les Demandes de Brevets Japonais publiées sous les n° 7-082132 et n° 7-102252, d'utiliser dans des compositions cosmétiques des hydrolysats préparés à partir de mucus d'huîtres, en tant qu'agents anti-oxydants propres à prévenir le vieillissement cutané et notamment l'apparition des rides. Ces hydrolysats sont obtenus en centrifugeant ou pressant des huîtres, après extraction de leurs coquilles et en éliminant la chair. Le mucus est alors soumis à une série de fractionnements par l'éthanol pour le débarrasser du chlorure de sodium qu'il renferme, puis à une protéolyse. Dans la Demande de Brevet Japonais n° 7-102252, le mucus, une fois hydrolysé, est soumis à une opération de dessalage complémentaire, toujours à l'aide d'éthanol, pour diminuer sa coloration.

Le coût de fabrication de tels hydrolysats est très élevé, notamment en raison des quantités non négligeables d'éthanol mises en jeu lors des opérations de dessalage et de la nécessité de disposer d'installations spécifiques et relativement onéreuses qu'impose l'utilisation de solvants organiques. De ce fait, indépendamment du point de savoir s'ils présentent une activité antiradicalaire significative, il n'est pas souhaitable d'utiliser ce type d'hydrolysats pour la fabrication à une échelle industrielle de compositions antiradicalaires, en particulier si ces dernières sont destinées à être commercialisées en tant que compléments alimentaires.

Or, dans le cadre de leurs travaux, les Inventeurs ont constaté que des hydrolysats d'huîtres obtenus en soumettant de la chair d'huîtres à l'action d'une protéase dans des conditions appropriées, présentent de manière surprenante une activité antiradicalaire encore plus élevée que celle observée pour les extraits aqueux d'huîtres testés par DUSSART dans l'étude précitée, et sont donc susceptibles d'être avantageusement utilisés pour la fabrication de compositions destinées à prévenir ou traiter les effets délétères des radicaux libres oxygénés.

La présente Invention a donc pour objet l'utilisation d'un hydrolysat enzymatique d'huîtres pour la préparation d'une composition pharmaceutique ayant une activité antiradicalaire, caractérisée en ce que ledit hydrolysat est obtenu par hydrolyse de chair d'huîtres au moyen d'une protéase selon un procédé ne mettant en oeuvre aucun solvant organique..

Selon une première disposition avantageuse de l'Invention, l'hydrolyse de la chair d'huîtres est réalisée par une protéase choisie parmi la subtilisine, la pepsine et la trypsine. En effet, outre que ces protéases ont un coût compatible avec une exploitation industrielle de l'Invention, elles présentent l'avantage de faire partie des enzymes dont l'utilisation est autorisée dans de très nombreux pays pour la préparation d'hydrolysats protéiques entrant dans la fabrication de compléments alimentaires.

Dans la mesure où les protéases ne sont pas toutes actives dans les mêmes gammes de pH et de température, les conditions de pH et de température dans lesquelles l'hydrolyse de la chair d'huîtres est effectuée dépendent de la protéase choisie pour réaliser cette hydrolyse.

De préférence, ces conditions de pH et de température sont telles qu'elles permettent d'obtenir une activité optimale de la protéase. Ainsi, par exemple, l'hydrolyse est conduite préférentiellement à un pH d'environ 8 et une température d'environ 60°C dans le cas de la subtilisine, à un pH d'environ 2 et une température d'environ 40°C dans le cas de la pepsine, et à un pH d'environ 8 et une température d'environ 37°C dans le cas de la trypsine.

Selon une autre disposition avantageuse de l'Invention, l'hydrolyse de la chair d'huîtres est effectuée pendant un temps suffisant pour que l'hydrolysat présente un degré d'hydrolyse protéique au moins égal à 30% et, de préférence, à 50%, ce degré d'hydrolyse protéique étant déterminé par la formule ci-après (ADLER-NISSEN, 1977, *Proc. Biochem.,* 12, 18-23) :

$$DH = (h/h \text{ total}) \times 100$$

dans laquelle :

- h total représente le nombre total de liaisons peptidiques présentes dans la chair d'huîtres au début de l'hydrolyse, tandis que
- h représente le nombre de liaisons peptidiques hydrolysées au cours de l'hydrolyse, et est déterminé par la différence entre le nombre d'extrémités aminées (ou carboxyliques) libres présentes dans l'hydrolysat au terme de l'hydrolyse ($h_1$) et le nombre d'extrémités aminées (ou carboxyliques) libres présentes dans le broyat au début de l'hydrolyse ($h_0$).

Au sens de la présente Invention, le début de l'hydrolyse correspond au moment où la protéase est mise en contact avec la chair d'huîtres, tandis que son terme correspond au moment où l'hydrolyse est arrêtée par inactivation de ladite protéase, par exemple par dénaturation thermique ou par modification du pH.

Le nombre total de liaisons peptidiques (h total) présentes dans la chair d'huîtres peut être obtenu par la différence entre la quantité d'acides aminés totaux (libres + liés) et la quantité d'acides aminés libres que renferme cette chair. Ces quantités d'acides aminés totaux et libres peuvent être déterminées, par exemple au moyen d'une trousse telle que celle commercialisée sous la marque WATERS AccQ-Tag Chemistry Package® par la Société WATERS. Le nombre de liaisons peptidiques hydrolysées (h) au cours de l'hydrolyse est, lui, obtenu par la différence entre la quantité d'extrémités aminées libres ($h_1$) présentes dans l'hydrolysat au terme de l'hydrolyse et la quantité d'extrémités aminées libres ($h_0$) présentes dans la chair d'huîtres au début de l'hydrolyse, lesquelles peuvent être déterminées, par exemple, par réaction avec du fluorodinitrobenzène selon le protocole décrit dans *Biochem. J.,* 45, 563, 1949.

Là également, le temps qu'il convient de laisser l'hydrolyse se faire afin d'obtenir un hydrolysat présentant un degré d'hydrolyse protéique au moins égal à 30% et, de préférence, à 50%, dépend de la protéase choisie pour réaliser cette hydrolyse, et, pour une même protéase, des conditions de pH, de température dans lesquelles l'hydrolyse est conduite, ainsi que de la dose à laquelle cette protéase est utilisée, l'hydrolyse s'effectuant, en effet, d'autant plus rapidement que la dose de protéase est plus élevée.

Selon encore une disposition avantageuse de l'Invention, l'hydrolysat est susceptible d'être obtenu par un procédé comprenant, préalablement à l'hydrolyse, une opération d'égouttage de la chair d'huîtres. Conformément à l'Invention, cette opération peut être réalisée en laissant simplement les huîtres, une fois extraites de leurs coquilles, reposer dans un égouttoir, de préférence à une température comprise entre 4 et 8°C pour prévenir toute altération de la chair, et ce, jusqu'à ce qu'il ne s'écoule plus de liquide dudit égouttoir.

Selon une disposition préférée de l'Invention, l'hydrolysat est susceptible d'être obtenu par un procédé comprenant, préalablement à l'hydrolyse, une opération de broyage de la chair d'huitres suivie éventuellement d'une opération de dilution dans l'eau du broyat résultant.

De manière particulièrement préférée, l'opération de broyage est réalisée après une opération d'égouttage de la chair d'huîtres.

Selon encore une autre disposition avantageuse de l'Invention, l'hydrolyse est arrêtée par dénaturation thermique de la protéase.

Selon encore une autre disposition avantageuse de l'Invention, l'hydrolysat est susceptible d'être obtenu par un procédé qui comprend, de plus, une opération de recueil de la phase liquide de l'hydrolysat tel qu'il se présente au terme de l'hydrolyse. Ce recueil, qui est destiné à éliminer les différents débris (débris de coquilles, débris membranaires, ...) susceptibles d'être présents dans cet hydrolysat, peut être réalisé par toutes les techniques classiquement utilisées pour séparer une phase liquide d'une phase solide telle que la centrifugation, l'ultracentrifugation, la filtration, la microfiltration, ces techniques pouvant être avantageusement combinées entre elles.

Selon une autre disposition préférée de l'Invention, l'hydrolysat est susceptible d'être obtenu par un procédé qui comprend les étapes suivantes :

   a) le broyage de chair d'huîtres préalablement égouttée,
   b) la dilution du broyat dans de l'eau, dans un rapport broyat/eau compris entre 30/70 et 70/30 (m/v), et, de préférence, entre 40/60 et 60/40 (m/v),
   c) l'hydrolyse du broyat ainsi dilué par de la subtilisine à un pH d'environ 8 et à une température d'environ 60°C, pendant un temps suffisant pour que l'hydrolysat présente un degré d'hydrolyse protéique au moins égal à 50%,

d) l'arrêt de l'hydrolyse par inactivation de la subtilisine, et
e) le recueil de la phase liquide de l'hydrolysat.

Selon encore une autre disposition préférée de l'Invention, l'hydrolysat est susceptible d'être obtenu par un procédé qui comprend les étapes suivantes :

a) le broyage de chair d'huîtres préalablement égouttée,
b) la dilution du broyat dans de l'eau, dans un rapport broyat/eau compris entre 30/70 et 70/30 (m/v), et, de préférence, entre 40/60 et 60/40 (m/v),
c) l'hydrolyse du broyat ainsi dilué par de la pepsine, à un pH d'environ 2 et à une température d'environ 40°C, pendant un temps suffisant pour que l'hydrolysat présente un degré d'hydrolyse protéique au moins égal à 50%,
d) l'arrêt de l'hydrolyse par inactivation de la pepsine, et
e) le recueil de la phase liquide de l'hydrolysat.

Selon encore une autre disposition préférée de l'Invention, l'hydrolysat est susceptible d'être obtenu par un procédé qui comprend les étapes suivantes :

a) le broyage de chair d'huîtres préalablement égouttée,
b) la dilution du broyat dans de l'eau, dans un rapport broyat/eau compris entre 30/70 et 70/30 (m/v), et, de préférence, entre 40/60 et 60/40 (m/v),
c) l'hydrolyse du broyat ainsi dilué par de la trypsine, à un pH d'environ 8 et à une température d'environ 37°C, pendant un temps suffisant pour que l'hydrolysat présente un degré d'hydrolyse protéique au moins égal à 50%,
d) l'arrêt de l'hydrolyse par inactivation de la trypsine, et
e) le recueil de la phase liquide de l'hydrolysat.

De tels hydrolysats enzymatiques d'huîtres présentent des propriétés antiradicalaires qui, outre d'être prononcées, sont extrêmement intéressantes puisqu'ils s'avèrent être capables, non seulement de neutraliser les effets des radicaux libres oxygénés produits au cours de réactions de peroxydation, mais également d'empêcher la formation de ces radicaux libres par ce qui apparaît être un mécanisme de chélation des métaux comme par exemple le cuivre, qui sont impliqués dans la genèse desdits radicaux libres.
De plus, ils présentent l'avantage de pouvoir être obtenus par un procédé simple à mettre en oeuvre et économiquement compatible avec les impératifs industriels, notamment en raison de ce qu'il ne requière l'utilisation d'aucun solvant organique.
Ces hydrolysats sont donc susceptibles d'être avantageusement utilisés pour la préparation :

- de compositions pharmaceutiques destinées à traiter les pathologies apparaissant liées à un déséquilibre entre la production et la destruction des radicaux libres oxygénés telles que précédemment évoquées,
- de compléments alimentaires propres à être utilisés, soit en tant qu'adjuvants à un traitement médical, soit à titre préventif, notamment par des personnes chez lesquelles il est souhaitable de renforcer les mécanismes naturels de défense contre les radicaux libres oxygénés, parce que ces moyens de défense sont physiologiquement diminués (personnes âgées, personnes souffrant de déficits alimentaires en vitamines et oligo-éléments, ...) ou parce que ces personnes sont amenées à se trouver dans des situations favorisant la formation excessive de radicaux libres oxygènes (exposition intense au soleil, exposition aux produits chimiques, ...), la protéase utilisée étant choisie parmi la pepsine, la subtilisine et la trypsine, ou encore
- de compositions cosmétiques visant à prévenir ou à traiter le vieillissement cutané dont l'origine est en grande partie liée aux radicaux libres générés au niveau de la peau par les rayonnements ultraviolets.

A cette fin, ils peuvent être utilisés soit tels quels, c'est-à-dire sous forme aqueuse ou éventuellement sous la forme de poudres sèches obtenues par exemple, par lyophilisation, soit en mélange avec des excipients physiologiquement acceptables et/ou d'autres substances actives et, notamment, des substances ayant également des propriétés antiradicalaires intrinsèques, et capables d'agir de manière synergique (vitamines A, C ou E, par exemple), au sein de formulations plus complexes.
Outre les dispositions qui précèdent, l'Invention comprend encore d'autres dispositions qui ressortiront du complément de description qui suit, qui se rapporte à des exemples illustrant l'activité hydrolytique d'enzymes sur des broyats de chair d'huitres, la préparation d'hydrolysats enzymatiques de chair d'huîtres ainsi que les propriétés biologiques de ces hydrolysats, et qui se réfère aux dessins annexés dans lesquels :

- la Figure 1 représente la cinétique de deux hydrolyses conduites sur des broyats de chair d'huîtres avec deux doses différentes de subtilisine ; tandis que

- la Figure 2 représente la cinétique d'une hydrolyse conduite sur un broyat de chair d'huîtres avec de la pepsine.

Il va de soi, toutefois, que ces exemples sont donnés uniquement à titre d'illustrations de l'objet de l'Invention et n'en constituent en aucune manière une limitation

EXEMPLE 1 : Etude de l'activité hydrolytique de la subtilisine sur des broyats de chair d'huîtres

Des huîtres creuses *Crassostrea gigas* vivantes, provenant de la station conchylicole expérimentale IFREMER de Bouin (VENDEE - FRANCE), après extraction de leurs coquilles, sont égouttées sur un tamis métallique pendant 1 heure à une température comprise entre 4 et 8°C, puis broyées pendant 2 minutes à 1 000 tr/minute à l'aide d'un Ultra-Turrax® (de puissance maximale égale à 170 W à 2 000 tr/minute).

Le broyat obtenu, après une éventuelle conservation à une température de -20°C et, dans ce cas, une décongélation, est introduit dans un réacteur. Il est additionné, sous agitation, de 60% (v/m) d'eau désionisée. Puis, on introduit dans le réacteur, toujours sous agitation, une dose de 14 UA (unités actives) ou de 38 UA de subtilisine (commercialisée sous la marque alcalase® 2.4L par la Société NOVO NORDISK) par kg du mélange qu'il contient. La température du réacteur est maintenue à 60°C pendant toute la durée de l'hydrolyse, soit pendant 4 heures. L'agitation est également maintenue et le pH est régulé à l'aide d'un pH-stat de manière à être constamment à une valeur de 8.

Au terme des 4 heures d'hydrolyse, l'activité de la subtilisine est arrêtée par dénaturation thermique de cette dernière, en plaçant le mélange réactionnel dans un bain-marie à 90°C pendant 25 minutes.

Des échantillons sont prélevés dans le réacteur, au moyen d'une pompe péristaltique, juste avant que n'y soit introduite la subtilisine ($t_0$), puis 15 et 30 minutes après l'introduction de cette enzyme dans le réacteur (soit à $t_{15}$ et $t_{30}$), puis toutes les 30 minutes et ce, jusqu'à l'arrêt de l'hydrolyse (soit à $t_{60}$, $t_{90}$, $t_{120}$, $t_{150}$, $t_{180}$, $t_{210}$ et $t_{240}$). Les échantillons qui renferment de la subtilisine sont placés dans un bain-marie à 90°C pendant 25 minutes de manière à stopper l'activité de cette dernière. Puis, tous les échantillons sont soumis à une centrifugation à 13 000 tr/minute. Les sumageants sont filtrés sur une membrane de 0,7 $\mu$m, puis sur une membrane de 0,16 $\mu$m.

L'activité hydrolytique de la subtilisine est appréciée en suivant :

- d'une part, l'évolution de la concentration des broyats en extrémités aminées libres entre $t_{15}$ et $t_{240}$, en dosant ces extrémités par réaction avec du fluorodinitrobenzène, ce suivi permettant d'établir la cinétique de l'hydrolyse, et
- d'autre part, l'évolution du degré d'hydrolyse protéique (DH) des broyats entre $t_{15}$ et $t_{240}$, ce degré d'hydrolyse protéique étant calculé selon la formule DH = (h/h total) x 100, dans laquelle h total est obtenu par le dosage des acides aminés totaux et libres présents dans les broyats à l'aide d'une trousse WATERS AccQ-Tag Chemistry Package®, tandis que h est déterminé par le dosage des extrémités aminées libres présentes dans les échantillons prélevés à $t_{15}$, $t_{30}$, ..., jusqu'à $t_{240}$ inclus, par réaction avec du fluorodinitrobenzène.

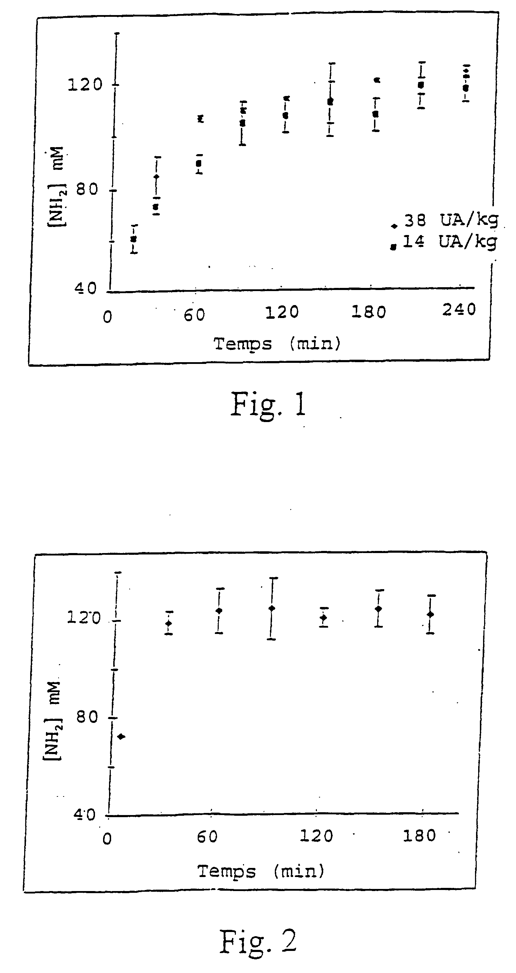

La Figure 1 représente la cinétique de l'hydrolyse conduite avec la dose de subtilisine de 14 UA/kg (■) et celle conduite avec la dose de subtilisine de 38 UA/kg (♦), les valeurs des concentrations en extrémités aminées libres étant exprimées en ordonnées et en mM, le temps étant exprimé en abscisses et en minutes.

Cette Figure montre que l'hydrolyse est plus rapide lorsque la dose de subtilisine est augmentée. Ainsi, le plateau est atteint au bout de 90 minutes d'hydrolyse pour la dose de 14 UA/kg, et ce délai est réduit à 60 minutes pour la dose de 38 UA/kg. Toutefois, la concentration en extrémités aminées libres pour laquelle le plateau est atteint est similaire pour les deux doses d'enzyme. Il en est de même de la concentration finale en extrémités aminées libres (environ 120 mM).

Le Tableau I ci-après présente les valeurs des degrés d'hydrolyse protéique (DH), exprimées en pourcentages, obtenues pour chacune des doses de subtilisine.

TABLEAU I

| Temps (minutes) | DH (%) | |
|---|---|---|
| | 14 UA/kg | 38 UA/kg |
| 15 | 14 | - |
| 30 | 23 | 31 |
| 60 | 34 | 46 |
| 90 | 45 | 48 |

TABLEAU I   (suite)

| Temps (minutes) | DH (%) | |
|---|---|---|
| | 14 UA/kg | 38 UA/kg |
| 120 | 47 | 51 |
| 150 | 50 | 51 |
| 180 | 47 | 56 |
| 210 | 54 | 54 |
| 240 | 54 | 58 |

Ce Tableau montre que, quelle que soit la dose de subtilisine utilisée, la vitesse d'hydrolyse diminue lorsque 45% des liaisons peptidiques potentiellement hydrolysables ont été rompues. L'hydrolyse se poursuit toutefois, mais de manière discrète, puisque les valeurs finales du degré d'hydrolyse protéique dépassent 50%, pour atteindre 54% dans un cas, et 58% dans l'autre cas.

**EXEMPLE 2 : Etude de l'activité hydrolytique de la pepsine sur des broyats de chair d'huîtres**

L'activité hydrolytique de la pepsine sur des broyats de chair d'huîtres est appréciée en utilisant un protocole opératoire identique à celui utilisé dans l'exemple 1, à ceci près que l'hydrolyse est conduite avec une dose de 1% en masse de pepsine rapportée à la masse totale du mélange broyat/eau désionisée présent dans le réacteur, à une température de 40°C et à un pH égal à 2.
La Figure 2 représente la cinétique de l'hydrolyse ainsi obtenue, les valeurs des concentrations en fonctions amines libres étant exprimées en ordonnées et en mM, le temps étant exprimé en abscisses et en minutes.
Cette Figure montre que l'hydrolyse s'effectue nettement plus rapidement que lorsqu'elle est conduite avec de la subtilisine, même à la dose de 38 UA/kg, puisque le plateau est atteint 30 minutes après l'introduction de la pepsine dans le réacteur. Toutefois, la concentration finale en fonctions amines libres dans l'hydrolysat, qui se situe aux environs de 120 mM, est tout à fait comparable à celle obtenue lorsque l'hydrolyse est conduite avec de la subtilisine.

**EXEMPLE 3 : Préparation d'hydrolysats enzymatiques de chair d'huîtres par utilisation de la subtilisine**

Sur la base des résultats obtenus dans l'étude objet de l'exemple 1, on prépare deux hydrolysats présentant des degrés d'hydrolyse protéique différents - qui seront dénommés ci-après respectivement hydrolysat A et hydrolysat B - en soumettant deux broyats de chair d'huîtres préalablement égouttée à une hydrolyse par de la subtilisine.
Les broyats de chair d'huîtres sont préparés et les hydrolyses sont conduites dans les mêmes conditions que celles décrites dans l'exemple 1, en utilisant une dose de subtilisine de 38 UA par kg de mélange broyat/eau désionisée.
Pour l'hydrolysat A, l'hydrolyse est arrêtée 4 heures après l'introduction de l'enzyme dans le réacteur, de manière à ce qu'il présente un degré d'hydrolyse protéique maximal, soit proche de 60%.
Pour l'hydrolysat B, l'hydrolyse est arrêtée 30 minutes après introduction de l'enzyme dans le réacteur, afin qu'il présente un degré d'hydrolyse protéique sensiblement égal à la moitié du degré d'hydrolyse protéique maximal, soit d'environ 30%.
Dans les deux cas, l'activité hydrolytique de la subtilisine est stoppée en plaçant les mélanges réactionnels dans un bain-marie à 90°C pendant 25 minutes. Les mélanges sont ensuite centrifugés à 4 000 tr/minute. Les surnageants sont filtrés sur une membrane de 0,7 μm, puis sur une membrane de 0,16 μm. Les hydrolysats ainsi préparés présentent un aspect granuleux de couleur brun-vert. Ils sont lyophilisés et placés dans des flacons à -20°C.

**EXEMPLE 4 : Caractérisation biochimique d'un hydrolysat enzymatique de chair d'huîtres obtenu conformément à l'Invention**

On réalise une étude visant à déterminer pour l'hydrolysat A préparé selon l'exemple 3 :

- sa teneur en matière sèche,
- sa teneur en matière minérale,
- sa teneur en protéines solubles,

- sa teneur en sucres totaux et en glycogène, ainsi que
- sa teneur et sa composition en acides aminés totaux et en acides aminés libres,

et à comparer les résultats avec ceux obtenus, dans les mêmes conditions, d'une part, pour un broyat de chair d'huîtres préparé comme décrit dans l'exemple 1 et d'autre part, pour un extrait aqueux d'huîtres préparé :

- en mélangeant un broyat de chair d'huîtres avec de l'eau désionisée (1/3, v/v) jusqu'à obtenir un mélange homogène, puis
- en soumettant le mélange résultant à une centrifugation à 3000 g pendant 20 minutes, et
- en lyophilisant le surnageant recueilli au terme de cette centrifugation.

La teneur en matière sèche est déterminée en plaçant des échantillons de l'hydrolysat A à une température de 100°C jusqu'à l'obtention d'un poids constant (6 heures minimum), et en calculant le pourcentage représenté par ce poids par rapport au poids initial de ces échantillons.

La teneur en matière minérale est déterminée en incinérant des échantillons de l'hydrolysat A à une température de 600°C pendant 12 heures, et en calculant le pourcentage représenté par le poids du résidu par rapport au poids de la matière sèche.

Les protéines solubles sont dosées à l'aide de la trousse commercialisée par la Société PIERCE sous la dénomination commerciale BCA® Protein Assay Reagent. L'albumine bovine est utilisée comme étalon.

Les sucres totaux et le glycogène sont dosés selon la méthode décrite par M. DUBOIS et *al.* (*Anal. Chem.*, 1956, 28, 350-356). Pour ces dosages, les échantillons sont prélablement délipidés selon la méthode de E. G. BLIGHT et W. J. DYER (*Can. J. Biochem. Physiol.*, 1959, 37, 911-917).

La teneur et la composition en acides aminés totaux et en acides aminés libres sont, quant à elles, déterminées à l'aide d'une trousse WATERS AccQ-Tag Chemistry Package®. Pour le dosage des acides aminés totaux, les échantillons de l'hydrolysat A sont préalablement soumis à une hydrolyse acide, par action d'HCI 6N pendant 12 heures, à 110°C et sous vide, tandis que, pour le dosage des acides aminés libres, les échantillons de l'hydrolysat A sont préalablement additionnés d'acide sulfo-salicilique et centrifugés de manière à provoquer une précipitation des protéines présentes dans ces échantillons.

Le Tableau II ci-après présente les teneurs en matière sèche, en matière minérale, en protéines solubles, en sucres totaux, en glycogène, en acides aminés totaux et en acides aminés libres présentées respectivement par l'hydrolysat A, le broyat d'huîtres et l'extrait aqueux d'huîtres.

Les teneurs en matière sèche sont exprimées en pourcentages par rapport au poids lyophilisé (% p/p) des échantillons, sauf dans le cas du broyat où la matière sèche est exprimée en pourcentage par rapport au poids frais (% p/p*) des échantillons. Les teneurs en matière minérale, en protéines solubles, en sucres totaux, en glycogène, en acides aminés totaux et en acides aminés libres sont exprimées en pourcentages par rapport aux poids sec (% p/p) des échantillons.

TABLEAU II

|  | Hydrolysat A | Broyat | Extrait aqueux |
|---|---|---|---|
| Matière sèche | 96,23 (% p/p) | 10,20 (%p/p*) | 95 (% p/p) |
| Matière minérale (% p/p) | 36,43 | 37,33 | 37 |
| Protéines solubles (% P/P) | 13,25 | 30 | 15 |
| Sucres totaux (% p/p) | 8,52 | 6,63 | 3,7 |
| Glycogène (% p/p) | 1,29 | 1 | 1,5 |
| Acides aminés totaux (% p/p) | 35,1 | 36,7 | 20,15 |

TABLEAU II   (suite)

|  | Hydrolysat A | Broyat | Extrait aqueux |
|---|---|---|---|
| **Acides aminés libres (% p/p)** | 17,8 | 7 | 8,10 |

Le Tableau III ci-après présente, lui, les compositions en acides aminés totaux et libres de l'hydrolysat A, du broyat d'huîtres et de l'extrait aqueux d'huîtres. Les teneurs de chaque acide aminé sont exprimées en pourcentages par rapport au poids total (% p/p) des acides aminés présents dans les échantillons.

**TABLEAU III**

| Acides aminés | HYDROLYSAT A | | BROYAT | | EXTRAIT AQUEUX | |
|---|---|---|---|---|---|---|
| | AA totaux (% p/p) | AA libres (% p/p) | AA totaux (% p/p) | AA libres (% p/p) | AA totaux (% p/p) | AA libres (% p/p) |
| Taurine | 10,67 | 19,25 | 11,48 | 55,98 | 30,47 | 60,66 |
| Hydroxyproline | – | – | – | – | – | – |
| Acide aspartique | 10,08 | 2,45 | 10,43 | 4,79 | 11,26 | 0,72 |
| Thréonine | 5,02 | 4,07 | 4,79 | – | 4,16 | – |
| Sérine | 4,67 | 6,58 | 5,02 | 2,28 | 4,46 | 2,21 |
| Acide glutamique | 13,26 | 8,08 | 13,49 | 8,60 | 11,91 | 11,46 |
| Proline | 5,12 | 2,20 | 4,85 | 7,59 | – | – |
| Glycine | 6,31 | 3,61 | 6,49 | 6,81 | 5,31 | 5,05 |
| Alanine | 5,64 | 6,43 | 4,39 | 3,21 | 5,80 | 8,01 |
| Cystéine | – | – | – | – | – | – |
| Valine | 4,51 | 4,94 | 4,16 | 0,42 | 2,77 | – |
| Méthionine | 2,13 | 2,76 | 2,06 | – | 1,53 | – |
| Isoleucine | 4,02 | 4,38 | 3,36 | – | 2,58 | – |
| Leucine | 6,18 | 7,63 | 6,32 | 0,65 | 4,66 | – |
| Tyrosine | 3,39 | 5,04 | 3,27 | – | 1,73 | 2,95 |
| Phénylalanine | 3,53 | 4,71 | 3,29 | 0,26 | 2,72 | – |
| Hydroxylysine | – | – | – | – | – | – |
| Lysine | 6,28 | 7,00 | 7,04 | 3,59 | 5,21 | 0,73 |
| Histidine | 2,33 | 2,92 | 2,92 | 1,70 | 1,24 | – |
| Arginine | 6,86 | 7,94 | 6,65 | 4,11 | 4,16 | 1,72 |

Le Tableau II montre que l'hydrolysat A présente une teneur en sucres totaux supérieure à celles retrouvées dans le broyat et dans l'extrait aqueux. Cet accroissement est dû à la déstructuration des tissus provoquée par l'hydrolyse enzymatique, permettant ainsi une plus forte solubilisation des sucres. La diminution de la teneur en protéines solubles que l'on observe entre le broyat et l'hydrolysat est une conséquence de l'hydrolyse des protéines natives. Cette hydrolyse génère une quantité importante de peptides et d'acides aminés libres qui réagissent peu avec le réactif utilisé pour le dosage des protéines solubles (BCA®). Par contre, la teneur en matières minérales ne varie pas entre les trois préparations.

Par ailleurs, il résulte du Tableau II que la teneur en acides aminés libres de l'hydrolysat A est notablement plus élevée que la teneur en acides aminés libres de l'extrait aqueux d'huîtres, cette dernière étant très proche de celle retrouvée pour le broyat de chair d'huîtres. L'augmentation de la quantité d'acides aminés libres présents dans l'hydrolysat A est directement liée à la rupture des liaisons peptidiques provoquée par la réaction d'hydrolyse.

Toutefois, au vu du Tableau III, il apparaît que la proportion de taurine libre, qui est connue pour présenter une activité anti-oxydante, est plus faible dans l'hydrolysat A que dans l'extrait aqueux d'huîtres. En effet, la taurine sous forme libre représente 60,66% des acides aminés libres dans l'extrait aqueux d'huîtres contre seulement 19,25% dans l'hydrolysat A.

**EXEMPLE 5 : Activité biologique des hydrolysats enzymatiques de chair d'huîtres obtenus conformément à l'Invention**

L'activité biologique des hydrolysats A et B préparés selon l'exemple 3 est appréciée par une série d'expérimentations visant à tester :

- d'une part, l'aptitude de ces hydrolysats à inhiber l'hémolyse induite par l'introduction d'un générateur de radicaux peroxydes, à savoir le 2,2'-azo-bis-(2-amidinopropane) dichlorhydrique (AAPH), dans une suspension d'hématies, et
- d'autre part, l'aptitude de ces hydrolysats à protéger les lipoprotéines de basse densité, plus connues sous la terminologie anglaise *"Low Density Lipoproteins"* (LDL) contre une oxydation induite par le cuivre.

**5.1 - Inhibition de l'hémolyse induite par l'AAPH :**

**a) Protocole :**

5 ml de sang humain sont prélevés sur tube EDTA (lequel est immédiatement placé dans de la glace pilée) et centrifugés pendant 10 minutes, à 1 000 g et à 4°C. Le plasma est éliminé et les hématies sont lavées 3 fois avec une solution de NaCl à 9‰ ou avec du tampon PBS (pH 7,4). 200 µl du culot cellulaire d'hématies sont ensuite dilués dans 9,8 µl de solution de NaCl à 9‰ ou de tampon PBS.

Dans un premier temps, la suspension globulaire obtenue est mise en contact pendant 10 minutes avec les solutions (NaCl 9‰ ou PBS) d'hydrolysats A ou B dont le volume est calculé de manière à ce que la solution finale corresponde à 25, 50 et 100 mg/l. Un échantillon sans hydrolysat constitue le témoin.

300 µl d'une solution d'AAPH ayant préalablement été mise à incuber à 37°C sont alors introduits dans les suspensions d'hématies et l'ensemble est disposé sous agitation douce au bain-marie pendant 40 minutes.

Un échantillon de la suspension d'hématies (sans AAPH ou produit) est parallèlement disposé pendant 1 heure à -80°C.

La lyse des hématies est appréciée en mesurant l'activité de la lactate déshydrogénase (LDH) à l'aide d'un automate HITACHI® 911. Chaque mesure est réalisée en double.

L'activité LDH déterminée sur les échantillons disposés à -80°C correspond à l'hémolyse totale des hématies.

L'activité LDH déterminée sur les échantillons ne contenant pas d'hydrolysat correspond à la sensibilité des hématies au *"stress radicalaire"* dans les conditions expérimentales. Cette mesure permet par ailleurs de vérifier que les conditions expérimentales (hémolyse < 100 %) sont convenables pour l'étude.

Pour chaque concentration d'hydrolysat, l'activité LDH est comparée à l'activité des échantillons ne contenant pas de produit et exprimée en pourcentage d'activité.

b) Résultats :

Le Tableau IV ci-après présente la moyenne des pourcentages d'inhibition (Ia) obtenus pour des solutions de 25, 50 et 100 mg/l d'hydrolysat A et d'hydrolysat B.

TABLEAU IV

| Concentration (mg/l) | Ia (%) | |
|---|---|---|
| | Hydrolysat A | Hydrolysat B |
| 25 | 38 | 25 |
| 50 | 74 | 48 |
| 100 | 96 | 98 |

Ce Tableau montre que les hydrolysats enzymatiques de chair d'huîtres obtenus conformément à l'Invention présentent une aptitude marquée à inhiber l'hémolyse induite par l'introduction d'un générateur de radicaux peroxydes au sein d'une suspension d'hématies - ce qui signifie qu'ils sont capables de neutraliser les effets oxydants de ces radicaux peroxydes -, puisque la concentration inhibitrice 50 ($IC_{50}$) de l'hydrolysat A est comprise entre 25 et 50 mg/l, tandis que celle de l'hydrolysat B s'élève à 50 mg/l.

A titre de comparaison, la concentration inhibitrice 50 ($IC_{50}$) obtenue par DUSSART (*ibid*) pour un extrait aqueux d'huîtres est de 275 mg/l.

5.2 - Protection des LDL contre une oxydation induite par le cuivre :

a) Protocole :

Les LDL sont préparées à partir de 100 ml de plasma (sang prélevé sur EDTA). Dans un premier temps, les VLDL sont éliminées par ultracentrifugation, 24 heures à 40 000 g (densité : 1,019). Une seconde ultracentrifugation, 24 heures à 40 000 g (densité : 1,063) permet l'obtention des LDL. Les LDL sont alors dialysées pendant 24 heures à 4°C contre du tampon TRIS-EDTA, aliquotées puis conservées à 4° C.

Les LDL (0,2 mg de protéines/ml de solution), préalablement dialysées contre du tampon PBS, sont mises à incuber 24 heures à 37°C en présence de cuivre (oxydant) et en présence ou non des produits étudiés.

Pour chaque étude, 3 déterminations sont donc réalisées parallèlement :

- LDL en absence de cuivre (témoin LDL natives),
- LDL en présence de 5 µM de sulfate de cuivre (témoin LDL oxydées),
- LDL en présence de 5 µM de sulfate de cuivre et de concentrations croissantes des hydrolysats A et B.

Après arrêt de l'oxydation par du BHT/EDTA, la solution de LDL est dialysée 24 heures à + 4°C et filtrée sur membrane *"millipore"* de 0,2 µm.

L'effet inhibiteur des hydrolysats vis à vis de l'oxydation des LDL par le cuivre est quantifié par le dosage de 2 marqueurs de lipoperoxydation :

- le MDA (malondialdéhyde), pour le calcul du pourcentage d'inhibition Ib,
- les hydroperoxydes, pour le calcul du pourcentage d'inhibition Ic.

. Dosage du MDA

Le MDA forme avec l'acide thiobarbiturique, à chaud et en milieu acide, un complexe chromogène fluorescent. Après extraction par le butanol normal, l'intensité de la fluorescence est mesurée à l'aide d'un spectrofluorimètre. Les concentrations de MDA sont déterminées au moyen d'une gamme de MDA s'étendant de 0,2 à 1 nmole.

. Dosage des hydroperoxydes

Les hydroperoxydes libèrent l'iode à partir d'une solution stabilisée d'iodure de potassium. L'iode libéré est mesuré par détermination de la densité optique (DO) à 365nm.

La concentration en iode de l'échantillon est ensuite calculée à partir du coefficient d'extinction $\varepsilon$ (=2,46 $10^4$, 1 cm, 1M) de cet élément.

b) <u>Résultats</u> :

Les Tableaux V et VI ci-après présentent respectivement les pourcentages d'inhibition (Ib) et (Ic) tels qu'obtenus pour des solutions de 25, 50, 100 et 250 mg/l d'hydrolysat A et d'hydrolysat B.

TABLEAU V

| Concentration (mg/l) | Ib (%) | |
|---|---|---|
| | Hydrolysat A | Hydrolysat B |
| 25 | -9 | 40 |
| 50 | 75 | 82 |
| 100 | 73 | 86 |
| 250 | 86 | 89 |

TABLEAU VI

| Concentration (mg/l) | Ic (%) | |
|---|---|---|
| | **Hydrolysat A** | **Hydrolysat B** |
| 25 | 10 | 71 |
| 50 | 100 | 100 |
| 100 | 100 | 100 |
| 250 | 100 | 100 |

Ces Tableaux montrent que les hydrolysats enzymatiques de chair d'huîtres obtenus conformément à l'Invention ont également une aptitude prononcée à s'opposer à une oxydation des LDL induite par le cuivre, aptitude qui pourrait être liée à un effet chélateur vis-à-vis des métaux.

**Revendications**

**1.** Utilisation d'un hydrolysat enzymatique d'huîtres pour la préparation d'une composition pharmaceutique ayant une activité antiradicalaire, **caractérisée en ce que** ledit hydrolysat est obtenu par hydrolyse de chair d'huîtres au moyen d'une protéase selon un procédé ne mettant en oeuvre aucun solvant organique.

**2.** Utilisation d'un hydrolysat enzymatique d'huîtres pour la préparation d'une composition cosmétique ayant une activité antiradicalaire, **caractérisé en ce que** ledit hydrolysat est obtenu par hydrolyse de chair d'huîtres au moyen d'une protéase selon un procédé ne mettant en oeuvre aucun solvant organique.

**3.** Utilisation d'un hydrolysat enzymatique d'huîtres pour la préparation d'un complément alimentaire ayant une activité antiradicalaire, **caractérisé en ce que** ledit hydrolysat est obtenu par hydrolyse de chair d'huîtres au moyen d'une protéase choisie parmi la subtilisine, la pepsine et la trypsine selon un procédé ne mettant en oeuvre aucun solvant organique.

**4.** Utilisation selon l'une quelconque des revendications 1 et 2, **caractérisée en ce que** l'hydrolyse est effectuée au moyen d'une protéase choisie parmi la subtilisine, la pepsine et la trypsine.

**5.** Utilisation selon l'une quelconque des revendications précédentes, **caractérisée en ce que** l'hydrolyse est effectuée pendant un temps suffisant pour que l'hydrolysat présente un degré d'hydrolyse protéique au moins égal à 30% et, de préférence, à 50%, ce degré d'hydrolyse protéique étant déterminé par la formule ci-après :

$$DH = (h/h\ total) \times 100$$

dans laquelle :

- h total représente le nombre total de liaisons peptidiques présentes dans la chair d'huîtres au début de l'hydrolyse, tandis que
- h représente le nombre de liaisons peptidiques hydrolysées au cours de l'hydrolyse, et est déterminé par la différence entre le nombre d'extrémités aminées libres présentes dans l'hydrolysat au terme de l'hydrolyse (h,) et le nombre d'extrémités aminées libres présentes dans la chair d'huîtres au début de l'hydrolyse (ho).

6. Utilisation selon l'une quelconque des revendications 1 à 5, **caractérisée en ce que** l'hydrolysat est susceptible d'être obtenu par un procédé comprenant, préalablement à l'hydrolyse, une opération d'égouttage de la chair d'huîtres.

7. Utilisation selon l'une quelconque des revendications 1 à 6, **caractérisée en ce que** l'hydrolysat est susceptible d'être obtenu par un procédé comprenant, préalablement à l'hydrolyse, une opération de broyage de la chair d'huîtres suivie éventuellement d'une opération de dilution dans l'eau du broyat résultant.

8. Utilisation selon la revendication 7, **caractérisée en ce que** l'opération de broyage est réalisée après une opération d'égouttage de la chair d'huîtres.

9. Utilisation selon l'une quelconque des revendications précédentes, **caractérisée en ce que** l'hydrolyse est arrêtée par dénaturation thermique de la protéase.

10. Utilisation selon l'une quelconque des revendications précédentes, **caractérisée en ce que** l'hydrolysat est susceptible d'être obtenu par un procédé comprenant, postérieurement à l'hydrolyse, une opération de recueil de la phase liquide de l'hydrolysat.

11. Utilisation selon l'une quelconque des revendications 5 à 10, **caractérisé en ce que** l'hydrolysat est obtenu par un procédé comprenant les étapes suivantes :

   a) le broyage de chair d'huîtres préalablement égouttée,
   b) la dilution du broyat dans de l'eau, dans un rapport broyat/eau compris entre 30/70 et 70/30 (m/v) et, de préférence, entre 40/60 et 60/40 (m/v),
   c) l'hydrolyse du broyat ainsi dilué par de la subtilisine, à un pH d'environ 8 et à une température d'environ 60°C, pendant un temps suffisant pour que l'hydrolysat présente un degré d'hydrolyse protéique au moins égal à 50%,
   d) l'arrêt de l'hydrolyse par inactivation de la subtilisine, et
   e) le recueil de la phase liquide de l'hydrolysat.

12. Utilisation selon l'une quelconque des revendications 5 à 10, **caractérisé en ce que** l'hydrolysat est obtenu par un procédé comprenant les étapes suivantes :

   a) le broyage de chair d'huîtres préalablement égouttée,
   b) la dilution du broyat dans de l'eau, dans un rapport broyat/eau compris entre 30/70 et 70/30 (m/v) et, de préférence, entre 40/60 et 60/40 (m/v),
   c) l'hydrolyse du broyat ainsi dilué par de la pepsine, à un pH d'environ 2 et à une température d'environ 40°C, pendant un temps suffisant pour que l'hydrolysat présente un degré d'hydrolyse protéique au moins égal à 50%,
   d) l'arrêt de l'hydrolyse par inactivation de la pepsine, et
   e) le recueil de la phase liquide de l'hydrolysat.

13. Utilisation selon l'une quelconque des revendications 5 à 10, **caractérisée en ce que** l'hydrolysat est obtenu par un procédé comprenant les étapes suivantes :

   a) le broyage de chair d'huîtres préalablement égouttée,
   b) la dilution du broyat dans de l'eau, dans un rapport broyat/eau compris entre 30/70 et 70/30 (m/v) et, de préférence, entre 40/60 et 60/40 (m/v),
c) l'hydrolyse du broyat ainsi dilué par de la trypsine, à un pH d'environ 8 et à une température d'environ 37°C, pendant un temps suffisant pour que l'hydrolysat présente un degré d'hydrolyse protéique au moins égal à 50%,
d) l'arrêt de l'hydrolyse par inactivation de la trypsine, et
e) le recueil de la phase liquide de l'hydrolysat.

**Patentansprüche**

1. Verwendung eines enzymatischen Hydrolysats aus Austern zur Herstellung einer Verbindung für pharmazeutische Zwecke, die gegen freie Radikale wirkt, **dadurch gekennzeichnet, dass** das Hydrolysat durch Hydrolyse von Austernfleisch mit Hilfe einer Protease gewonnen wird, nach einem Verfahren, für das keine organischen Lösemittel verwendet werden.

2. Verwendung eines enzymatischen Hydrolysats aus Austern zur Herstellung einer Verbindung für kosmetische Zwekke, die gegen freie Radikale wirkt, **dadurch gekennzeichnet, dass** das Hydrolysat durch Hydrolyse von Austernfleisch mit Hilfe einer Protease gewonnen wird, nach einem Verfahren, für das keine organischen Lösemittel verwendet werden.

3. Verwendung eines enzymatischen Hydrolysats aus Austern zur Herstellung eines Nahrungsergänzungsmittels, das gegen freie Radikale wirkt, **dadurch gekennzeichnet, dass** das Hydrolysat durch Hydrolyse von Austernfleisch mit Hilfe einer Protease gewonnen wird, die unter Subtilisin, Pepsin und Trypsin ausgewählt wird, nach einem Verfahren, für das keine organischen Lösemittel verwendet werden.

4. Verwendung nach einem der Ansprüche 1 und 2, **dadurch gekennzeichnet, dass** die Hydrolyse mit Hilfe einer Protease ausgeführt wird, die unter Subtilisin, Pepsin und Trypsin ausgewählt wird.

5. Verwendung nach einem der vorhergehenden Ansprüche, **dadurch gekennzeichnet, dass** die Hydrolyse ausreichend lange ausgeführt wird, damit das Hydrolysat einen Hydrolysegrad der Eiweiße von mindestens 30% und vorzugsweise 50% aufweist, wobei der Hydrolysegrad der Eiweiße nach folgender Formel bestimmt wird:

$$HG = (h/h \text{ gesamt}) \times 100$$

wobei :

- h gesamt die Gesamtzahl der Peptidbindungen im Austernfleisch zu Beginn der Hydrolyse ist, während
- h die Anzahl der während der Hydrolyse hydrolysierten Peptidbindungen ist und bestimmt wird durch den Unterschied zwischen der Zahl der freien Aminoenden im Hydrolysat am Ende der Hydrolyse ($h_1$) und der Zahl der freien Aminoenden im Austernfleisch zu Beginn der Hydrolyse ($h_0$) .

6. Verwendung nach einem der Ansprüche 1 bis 5, **dadurch gekennzeichnet, dass** das Hydrolysat dazu geeignet ist, durch ein Verfahren gewonnen zu werden, das vor der Hydrolyse das Abtropfen des Austernfleischs beinhaltet.

7. Verwendung nach einem der Ansprüche 1 bis 6, **dadurch gekennzeichnet, dass** das Hydrolysat dazu geeignet ist, durch ein Verfahren gewonnen zu werden, das vor der Hydrolyse die Zerkleinerung des Austernfleischs beinhaltet, bevor die entstehende zerkleinerte Masse gegebenenfalls in Wasser verdünnt wird.

8. Verwendung nach Anspruch 7, **dadurch gekennzeichnet, dass** die Zerkleinerung nach dem Abtropfen des Austernfleischs ausgeführt wird.

9. Verwendung nach einem der vorhergehenden Ansprüche, **dadurch gekennzeichnet, dass** die Hydrolyse durch Hitzedenaturierung der Protease beendet wird.

10. Verwendung nach einem der vorhergehenden Ansprüche, **dadurch gekennzeichnet, dass** das Hydrolysat dazu geeignet ist, durch ein Verfahren gewonnen zu werden, das nach der Hydrolyse die Gewinnung der flüssigen Phase des Hydrolysats umfasst.

**11.** Verwendung nach einem der Ansprüche 5 bis 10, **dadurch gekennzeichnet, dass** das Hydrolysat durch ein Verfahren gewonnen wird, das folgende Schritte umfasst:

a) Zerkleinern des vorher abgetropften Austernfleischs,
b) Verdünnen der zerkleinerten Masse in Wasser, in einem Verhältnis zerkleinerte Masse/Wasser zwischen 30/70 und 70/30 (m/v) und vorzugsweise zwischen 40/60 und 60/40 (m/v),
c) Hydrolyse der so verdünnten zerkleinerten Masse durch Subtilisin, bei einem pH-Wert von ungefähr 8 und bei einer Temperatur von ungefähr 60°C, ausreichend lange, damit das Hydrolysat einen Hydrolysegrad der Eiweiße von mindestens 50% aufweist,
d) Beenden der Hydrolyse durch Inaktivierung des Subtilisins, und
e) Gewinnen der flüssigen Phase des Hydrolysats.

**12.** Verwendung nach einem der Ansprüche 5 bis 10, **dadurch gekennzeichnet, dass** das Hydrolysat durch ein Verfahren mit folgenden Schritten gewonnen wird:

a) Zerkleinern des vorher abgetropften Austernfleischs,
b) Verdünnen der zerkleinerten Masse in Wasser, in einem Verhältnis zerkleinerte Masse/Wasser zwischen 30/70 und 70/30 (m/v) und vorzugsweise zwischen 40/60 und 60/40 (m/v),
c) Hydrolyse der so verdünnten zerkleinerten Masse durch Pepsin, bei einem pH-Wert von ungefähr 2 und bei einer Temperatur von ungefähr 40°C, ausreichend lange, damit das Hydrolysat einen Hydrolysegrad der Eiweiße von mindestens 50% aufweist,
d) Beenden der Hydrolyse durch Inaktivierung des Pepsins, und
e) Gewinnen der flüssigen Phase des Hydrolysats.

**13.** Verwendung nach einem der Ansprüche 5 bis 10, **dadurch gekennzeichnet, dass** das Hydrolysat durch ein Verfahren mit folgenden Schritten gewonnen wird:

a) Zerkleinern des vorher abgetropften Austernfleischs,
b) Verdünnen der zerkleinerten Masse in Wasser, in einem Verhältnis zerkleinerte Masse/Wasser zwischen 30/70 und 70/30 (m/v) und vorzugsweise zwischen 40/60 und 60/40 (m/v),
c) Hydrolyse der so verdünnten zerkleinerten Masse durch Trypsin, bei einem pH-Wert von ungefähr 8 und bei einer Temperatur von ungefähr 37°C, ausreichend lange, damit das Hydrolysat einen Hydrolysegrad der Eiweiße von mindestens 50% aufweist,
d) Beenden der Hydrolyse durch Inaktivierung des Trypsins, und
e) Gewinnen der flüssigen Phase des Hydrolysats.

**Claims**

**1.** Use of an oyster enzymatic hydrolysate in the preparation of a pharmaceutical composition having free-radical scavenging activity, **characterised in that** said hydrolysate is produced by hydrolysis of oyster flesh by means of a protease according to a method which does not use any organic solvent.

**2.** Use of an oyster enzymatic hydrolysate in the preparation of a cosmetic composition having free-radical scavenging activity, **characterised in that** the hydrolysate is produced by hydrolysis of oyster flesh by means of a protease according to a method which does not use any organic solvent.

**3.** Use of an oyster enzymatic hydrolysate in the preparation of a nutritional complement having free-radical scavenging activity, **characterised in that** the hydrolysate is produced by hydrolysis of oyster flesh by means of a protease selected from subtilisin, pepsin and trypsin according to a method which does not use any organic solvent.

**4.** Use according to either claim 1 or claim 2, **characterised in that** hydrolysis is carried out by means of a protease selected from subtilisin, pepsin and trypsin.

**5.** Use according to any one of the preceding claims, **characterised in that** hydrolysis is carried out for a period of time such that the hydrolysate has a degree of protein hydrolysis of at least 30% and preferably of 50%, this degree of protein hydrolysis being determined by the following formula:

$$DH = (h/h\ total) \times 100$$

where:

- h total represents the total number of peptide linkages present in the oyster flesh at the start of hydrolysis, whereas
- h represents the number of hydrolysed peptide linkages during hydrolysis and is determined by the difference between the number of free amino-containing ends present in the hydrolysate at the end of hydrolysis ($h_1$) and the number of free amino-containing ends present in the oyster flesh at the start of hydrolysis ($h_0$).

6. Use according to any one of claims 1 to 5, **characterised in that** the hydrolysate can be produced by a method comprising, before hydrolysis, an operation for draining the oyster flesh.

7. Use according to any one of claims 1 to 6, **characterised in that** the hydrolysate can be produced by a method comprising, before hydrolysis, an operation for grinding the oyster flesh, optionally followed by an operation for diluting the resultant ground product in water.

8. Use according to claim 7, **characterised in that** the grinding operation is carried out after an operation for draining the oyster flesh.

9. Use according to any one of the preceding claims, **characterised in that** hydrolysis is terminated by thermal denaturation of the protease.

10. Use according to any one of the preceding claims, **characterised in that** the hydrolysate can be produced by a method comprising, after hydrolysis, an operation for collecting the liquid phase of the hydrolysate.

11. Use according to any one of claims 5 to 10, **characterised in that** the hydrolysate is produced by a method comprising the following steps:

    a) grinding oyster flesh which has been drained beforehand,
    b) diluting the ground product in water at a ground product/water ratio of between 30/70 and 70/30 (m/v) and preferably of between 40/60 and 60/40 (m/v),
    c) hydrolysis of the thus diluted ground product by subtilisin, at a pH of approximately 8 and at a temperature of approximately 60°C for a time such that the hydrolysate has a degree of protein hydrolysis of at least 50%,
    d) terminating hydrolysis by inactivating the subtilisin and
    e) collecting the liquid phase of the hydrolysate.

12. Use according to any one of claims 5 to 10, **characterised in that** the hydrolysate is produced by a method comprising the following steps:

    a) grinding oyster flesh which has been drained beforehand,
    b) diluting the ground product in water at a ground product/water ratio of between 30/70 and 70/30 (m/v) and preferably of between 40/60 and 60/40 (m/v),
    c) hydrolysis of the thus diluted ground product by pepsin at a pH of approximately 2 and at a temperature of approximately 40°C, for a time such that the hydrolysate has a degree of protein hydrolysis of at least 50%,
    d) terminating hydrolysis by inactivating the pepsin and
    e) collecting the liquid phase of the hydrolysate.

13. Use according to any one of claims 5 to 10, **characterised in that** the hydrolysate is produced by a method comprising the following steps:

    a) grinding oyster flesh which has been drained beforehand,
    b) diluting the ground product in water at a ground product/water ratio of between 30/70 and 70/30 (m/v) and preferably of between 40/60 and 60/40 (m/v),
    c) hydrolysis of the thus diluted ground product by trypsin at a pH of approximately 8 and at a temperature of approximately 37°C for a time such that the hydrolysate has a degree of protein hydrolysis of at least 50%,
    d) terminating hydrolysis by inactivating the trypsin and

e) collecting the liquid phase of the hydrolysate.

Fig. 1

Fig. 2